# EUROPÄISCHE PATENTANMELDUNG

(11) **EP 2 179 988 A1**
(43) Veröffentlichungstag der Anmeldung: **28.04.2010**
(21) Anmeldenummer: 09173002.8
(22) Anmeldetag: 14.10.2009
(51) Int. Cl.: C07D 211/02, C07D 211/46

(54) **Herstellung von (N-Heterozyklyl)-Arylethern**

(30) Priorität: 25.10.2008 DE 102008053240
(71) Anmelder: Saltigo GmbH, 40764 Langenfeld (DE)
(72) Erfinder: KREIS, Michael, 51373, Leverkusen (DE)
(74) Vertreter: Wichmann, Birgid

(57) **Zusammenfassung**

Die vorliegende Erfindung betrifft ein Verfahren zur Herstellung von gegebenenfalls substituierten (N-Heterozyklyl)-Arylethem.

## Beschreibung

Die vorliegende Erfindung betrifft ein Verfahren zur Herstellung von gegebenenfalls substituierten (N-Heterozyklyl)-Arylethern.

Diverse (N-Heterozyklyl)-Arylether besitzen bereits eine Bedeutung als Intermediate für die Synthese von Arzneimitteln und Agrochemikalien und stellen ein wesentliches Strukturelement in Wirkstoffen gegen z.B. Infektionskrankheiten, Schizophrenie, Depressionen, neurodegenerativen Krankheiten sowie in Pestizidintermediaten dar.

Verschiedene Verfahren zur Herstellung von (N-Heterozyklyl)-Arylethern sind bekannt.

So wird beispielsweise in der WO 2005/092832 die Herstellung von (N-Heterozyklyl)-Arylethem, insbesondere eines Arylpiperidinylethers, ausgehend von N-Boc-4-hydroxypiperidinol über eine aufwändige drei Stufen-Synthese beschrieben. Gleiches gilt für die WO 97/23216.

Alternativ dazu erfolgt in der EP 1947098 A1 die Synthese ausgehend von N-Benzyl-4-hydroxypiperidinol unter Einführung einer Schutzgruppe und Umsetzung mit 4-Trifluormethylfluorbenzol, was deutliche Nachteile in der Wirtschaftlichkeit aufweist.

Die Herstellung von einem (N-Heterozyklyl)-Arylether wird in der WO 2002/072621 ausgehend von N-Boc-4-hydroxypiperidinol über eine Mitsunobu-Reaktion unter Verwendung von Diethylazocarboxylat (DEAD) beschrieben. Dieser Weg ist aufgrund der Explosivität des eingesetzten DEAD schwierig zu realisieren, insbesondere im großtechnischen Maßstab.

Die bekannten Verfahren weisen die Nachteile auf, dass sie entweder unökonomisch, ökologisch bedenklich oder sicherheitstechnisch problematisch sind.

Es bestand daher die Aufgabe, ein Verfahren bereitzustellen, das die Nachteile der bisherigen Verfahren nicht aufweist und die (N-Heterozyklyl)-Arylether in guten Ausbeuten, kostengünstig in technischem Maßstab liefert.

Überraschenderweise wurde gefunden, dass mit dem erfindungsgemäßen Verfahren, unter Verwendung von Übergangsmetall-Katalysatoren und spezieller Lösungsmittel, insbesondere von Carbonsäuren, die Nachteile des Standes der Technik beseitigt werden konnten und (N-Heterozyklyl)-Arylether in guten Ausbeuten hergestellt werden konnten.

Gegenstand der Erfindung ist daher ein Verfahren zur Herstellung von Verbindungen der
Formel (I)

[R²-O]-ZYKLYL-N-R¹ (I)

**dadurch gekennzeichnet, dass** Verbindungen der Formel (II)

[R²-O]-ARYL-N (II)

in Gegenwart von mindestens einem Übergangsmetall-Katalysator,
mindestens einem organischen polaren Lösungsmittel
und Wasserstoff
umgesetzt werden,
wobei ZYKLYL-N für einen gegebenenfalls substituierten 5- bis 8- gliedrigen, zyklischen gesättigten oder teilweise ungesättigten, nicht aromatischen, N-heterozyklischen Rest, mit bis zu zwei Stickstoffatomen steht, der über ein Ringkohlenstoffatom mit dem Sauerstoffatom des [R²-O]-Restes verknüpft ist, und ARYL-N für einen gegebenenfalls substituierten N-heterozyklischen 5- bis 8- gliedrigen aromatischen Rest, mit bis zu zwei Stickstoffatomen steht, der über ein Ringkohlenstoffatom des aromatischen Restes mit dem Sauerstoffatom des [R²-O]-Restes verknüpft ist,
R² für ein gegebenenfalls substituiertes C₆-C₂₀-Aryl steht und
R¹ mit einem Stickstoffatom des ZYKLY-N verbunden ist und Wasserstoff oder -C(=O)(R) darstellt und R für C₁-C₁₅-Alkyl, besonders bevorzugt C₁-C₆-Alkyl, C₁-C₈-Alkylthio, C₁-C₈-Alkoxy, C₆-C₂₄-Aryl, C₈-C₂₆-Arylalkyl, C₁-C₈-Halogenalkyl, C₁-C₈-Halogenalkoxy, C₁-C₈-Halogenalkylthio, mit Halogen = F, Cl, Br, 5- bis 8- gliedriges gesättigtes Heterozyklyl mit Hetero = (Sauerstoff, Schwefel oder Stickstoff), Wasserstoff oder C₁-C₈-Mono- bzw. C₁-C₁₆-Dialkylamino, die gegebenenfalls weiter substituiert sein können, steht, insbesondere bevorzugt ist R C₁-C₆-Alkyl oder Wasserstoff.

Alkyl bzw. Alkoxy steht im Rahmen der Erfindung für einen geradkettigen, zyklischen, verzweigten oder unverzweigten Alkyl- bzw. Alkoxy- Rest mit 1 bis 15 (C₁-C₁₅), bevorzugt 1 bis 12 (C₁-C₁₂), besonders bevorzugt 1 bis 6 (C₁-C₆) und ganz besonders bevorzugt mit 3 bis 6 (C₃-C₆) Kohlenstoffatomen.

Beispielhaft und vorzugsweise steht Alkyl für Methyl, Ethyl, n-Propyl, Isopropyl, n-, i-, s- oder t-Butyl, Cyclopropyl, Cyclobutyl, Cyclopentyl, Cyclohexyl, Cycloheptyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, n-Heptyl, n-Octyl, n-Decyl und n-Dodecyl und Alkoxy für Methoxy, Ethoxy, n-Propoxy, Isopropoxy, t-Butoxy, n-Pentoxy und n-Hexoxy.

Bevorzugt steht R² für einen C₆-C₂₀-Arylrest, der gegebenenfalls substituiert ist, mit Alkyl, Aryl, Alkoxy, Halogenalkyl, Halogenalkylthio oder Halogenalkyloxy mit Halogen = F, Cl und Br.

Besonders bevorzugt steht R² für einen C₆-C₂₀-Arylrest, der gegebenenfalls einfach oder mehrfach mit (C₁-C₆)-Alkyl, (C₁-C₆)-Alkoxy, (C₁-C₇)-Perfluoralkyl, (C₁-C₇)-Perchloralkyl, (C₁-C₇)-Perfluoralkoxy und (C₁-C₇)-Perchloralkoxy substituiert ist.

In einer besonders bevorzugten Ausführungsform steht R² für einen C₆-C₂₀-Arylrest, der gegebenenfalls einfach oder mehrfach mit Trifluormethoxy, Methoxy und/oder Methyl substituiert ist. In einer ganz besonders bevorzugten Ausführungsform steht R² für 2-, 3-, oder 4-Trifluormethoxyphenyl, 2-, 3-, oder 4-Methoxyphenyl oder 2-, 3-, oder 4-Phenylmethyl.

Aryl steht im Rahmen der Erfindung für einen mono-, bi- oder trizyklischen carbozyklischen aromatischen Rest mit vorzugsweise 6 bis 20 aromatischen Kohlenstoffatomen (C₆-C₂₀-Aryl).

Die carbozyklischen aromatischen Reste können mit bis zu fünf gleichen oder verschiedenen Substituenten pro Zyklus substituiert sein, wie z.B. Alkyl, Alkoxy, Aryl, Arylalkyl, Carboxyl, Dialkylamino, Halogen, Halogenalkyl, Halogenalkoxy, Halogenalkylthio und Monoalkylamino. Beispielhaft und bevorzugt steht C₆-C₂₀-Aryl für Biphenyl, Phenyl, Naphthyl, Phenanthrenyl, Anthracenyl oder Fluorenyl.

Mono- bzw. Dialkylamino stehen im Rahmen der Erfindung für eine Amino-Gruppe mit einem oder zwei gleichen oder verschiedenen, zyklischen, geradkettigen oder verzweigten Alkylsubstituenten, die vorzugsweise jeweils 1 bis 10, besonders bevorzugt 1 bis 6 (C₁-C₆) Kohlenstoffatome aufweisen.

Beispielhaft und vorzugsweise steht Monoalkylamino für Methylamino, Ethylamino, n-Propylamino, Isopropylamino, t-Butylamino, n-Pentylamino und n-Hexylamino sowie Dialkylamino für *N*,*N*-Dimethyl-amino, *N,N-*Diethylamino, *N*-Ethyl-*N*-methylamino, *N*-Methyl-*N*-n-propylamino, *N*-Isopropyl-*N*-n-propylamino, *N*-t-Butyl-*N*-methylamino, *N*-Ethyl-*N*-n-pentylamino und *N*-n-Hexyl-*N*-methylamino.

Arylalkyl bedeutet jeweils unabhängig voneinander einen geradkettigen, zyklischen, verzweigten oder unverzweigten Alkyl-Rest nach vorstehender Definition, der einfach, mehrfach oder vollständig durch Aryl-Reste gemäß vorstehender Definition substituiert sein kann. Ein Beispiel für Arylalkyl ist Benzyl. Bevorzugt sind Arylalkyle mit 7 bis 13 (C₇-C₁₃) Kohlenstoffatome, besonders bevorzugt sind Arylalkyle mit 7 bis 10 (C₇-C₁₀) Kohlenstoffatomen.

Halogene im Rahmen der Erfindung sind bevorzugt Fluor, Chlor und Brom, besonders bevorzugt Fluor und Chlor.

Halogenalkyl bzw. Halogenalkoxy steht im Rahmen der Erfindung für einen geradkettigen, zyklischen, verzweigten oder unverzweigten Alkyl-, bzw. Alkoxy-Rest nach vorstehender Definition, der einfach, mehrfach oder vollständig mit Halogenatomen substituiert ist.

Beispielhaft und vorzugsweise steht Halogenalkyl für Chlorethyl, Chlorpropyl, Dichlormethyl, Difluormethyl, Fluormethyl, Fluorethyl, Fluorpropyl und 2,2,2-Trifluorethyl und Halogenalkoxy für Difluormethoxy, Fluorethoxy, Fluormethoxy, Trifluormethoxy, Trichlormethoxy und 2,2,2-Trifluorethoxy.

Halogenalkyl bzw. Halogenalkoxy beinhalten beispielsweise die Perfluoralkyl-, Perchloralkyl- bzw. die Perfluoralkoxy- und Perchloralkoxy-Reste. Besonders bevorzugt sind Perfluoralkyl-, Perchloralkyl- bzw. Perfluoralkoxy- und Perchloralkoxy-Reste mit 1 bis 5 (C₁-C₅) Kohlenstoffatomen. Ganz besonders bevorzugt sind die Perfluoralkoxy- und Perchloralkoxy -Reste ausgewählt aus der Gruppe Trifluormethyl, Trichlormethyl, Pentafluorethyl, Heptafluorisopropyl und Nonafluorbutyl.

In einer weiteren bevorzugten Ausführungsform steht ARYL-N für Pyridyl, das gegebenenfalls einfach oder mehrfach mit Alkoxy, Alkyl, Aryl, Halogenalkyl, Halogenalkylthio oder Halogenalkyloxy mit Halogen = F, Cl und Br substituiert ist.

Besonders bevorzugt steht ARYL-N für Pyridyl, das gegebenenfalls einfach oder mehrfach mit (C₁-C₆)-Alkyl, (C₁-C₆)-Alkoxy, (C₁-C₇)-Perfluoralkyl, (C₁-C₇)-Perchloralkyl, (C₁-C₇)-Perfluoralkoxy und (C₁-C₇)-Perchloralkoxy substituiert ist.

Halogenallcylthio bedeutet im Rahmen der Erfindung einen geradkettigen, zyklischen, verzweigten oder unverzweigten Rest mit 1 bis 15 Kohlenstoffatomen, bevorzugt mit 1 bis 7 (C₁-C₇) Kohlenstoffatomen, der einfach, mehrfach oder vollständig mit Halogenatomen substituiert ist. Beispielhaft und vorzugsweise steht Halogenalkylthio für Chlorethylthio, Chlorbutylthio, Chlorhexylthio, Chlorpentylthio, Chlordodecylthio, Dichlorethylthio, Fluorethylthio, Trifluormethylthio und 2,2,2-Trifluorethylthio.

In einer ganz besonders bevorzugten Ausführungsform steht ARYL-N für 2-,3-, oder 4-Trifluormethoxypyridyl, 2-,3-, oder 4-Methoxypyridyl oder 2-,3-, oder 4-Methylpyridyl.

N-heterozyklischer 5- bis 8-gliedriger Rest steht im Rahmen der Erfindung vorzugsweise für den Rest eines aromatischen und/oder nicht aromatischen Heterozykluses , mit bis zu zwei Stickstoffatomen, vorzugsweise einem Stickstoffatom, der über ein Ringkohlenstoffatom mit dem Sauerstoffatom des [R²-O]-Restes verknüpft ist. Beispielhaft und besonders bevorzugt seien genannt: Azepinyl, Pyrrolyl, Imidazolyl, Pyridyl, Pyrimidinyl, Azepanyl, Pyrrolidinyl, Piperidinyl, Piperazinyl und Pyridazinyl.

5- bis 8- gliedriges gesättigtes Heterozyklyl steht im Rahmen der Erfindung vorzugsweise für einen Heterozyklus-Rest, mit bis zu 3 gleichen oder verschiedenen Heteroatomen aus der Reihe S, N und/oder O, der über ein Ringkohlenstoffatom, Ringstickstoffatom, Ringsauerstoffatom oder Ringschwefelatom verknüpft ist. Bevorzugt ist ein 5- bis 8-gliedriges gesättigtes Heterozyklyl mit bis zu 2 gleichen oder verschiedenen Heteroatomen aus der Reihe S, N und/oder O. Beispielhaft seien genannt: Azepanyl, Pyrrolidinyl, Piperidinyl, Piperazinyl, Morpholinyl oder Tetrahydrofuryl. Bevorzugt sind Azepanyl, Pyrrolidin-2-yl, Pyrrolidin-3-yl, Pyrrolidin-1-yl, Piperidin-1-yl, Piperidin-4-yl, Piperazinyl, Morpholinyl, Tetrahydrofur-2-yl oder Tetrahydrofur-3-yl.

Beispielsweise und vorzugsweise werden als Verbindungen der Formel (II) unsubstituierte Phenoxypyridine, 4-[4-R²-O]-ARYL-N oder 4-[2- R²-O]-ARYL-N eingesetzt, d.h. im Besonderen 4-[4-Trifluormethoxyphenoxy]pyridin, 4-[4-Methylphenoxy]pyridin, 4-Phenoxypyridin, 4-[4-Methoxyphenoxy]pyridin und/oder 4-[2-Trifluormethoxyphenoxy]pyridin.

In einer weiteren besonders bevorzugten Ausführungsform sind die Verbindungen der Formel (I) 4-[4-Trifluormethoxyphenoxy]piperidin, 4-[4-Methylphenoxy]piperidin, 4-Phenoxypiperidin, 4-[4-Methoxyphenoxy]piperidin, 4-[2-Trifluormethoxyphenoxy]piperidin, 4-[4-Trifluormethoxyphenoxy]-N-piperidinylacetamid, 4-[4-Methylphenoxy]-N-piperidinylacetamid, 4-Phenoxy-N-piperidinylacetamid, 4-[4-Methoxyphenoxy]-N-piperidinylacetamid und/oder 4-[2-Trifluormethoxyphenoxy]-N-piperidinylacetamid.

In einer weiteren bevorzugten Ausführungsform der Erfindung ist -C(=O)(R) ein Aryl-Rest.

Der Rahmen der Erfindung umfasst alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

Als Lösungsmittel im Sinne der Erfindung können eingesetzt werden organische, polare Lösungsmittel aliphatische oder alizyklische Ketone, Ether, Ester, Anhydride, Carbonsäuren, Alkohole und/oder Wasser. In einer bevorzugten Ausführungsform des erfindungsgemäßen Verfahrens handelt es bei dem organischen polaren Lösungsmittel um aliphatische oder alizyklische Ketone, Ether, Anhydride, Ester und/oder Mono-, Di- oder Tricarbonsäuren.

Besonders bevorzugt handelt es sich bei den organischen, polaren Lösungsmitteln um Ameisensäure, Essigsäure, Propionsäure, n-, t-, i-Butansäure, Isopropansäure, Pentansäure, Butandisäure, Oxalsäure, Zitronensäure, Pyrrolidin-2-carbonsäure oder Mischungen dieser Säuren.

Ganz besonders bevorzugt sind die organischen, polaren Lösungsmitteln Ameisensäure, Essigsäure oder Propionsäure oder Mischungen dieser Säuren.

In einer weiteren bevorzugten Ausführungsform der Erfindung ist das Übergangsmetall des Übergangsmetall-Katalysators Rhodium, Ruthenium, Iridium, Platin oder Palladium und deren Salze, besonders bevorzugt Rhodium, Ruthenium, Platin oder Palladium und deren Salze.

In einer anderen Ausführungsform des erfindungsgemäßen Verfahrens befindet sich das Übergangsmetall bevorzugt auf einem Träger, ausgewählt aus der Gruppe Siliziumdioxid, Aluminiumoxid, Titandioxid, Zeolithe oder Kohlenstoff. Ganz besonders bevorzugt handelt es sich bei dem Träger um Aktivkohle oder Titandioxid.

In den Fällen, in denen die Übergangsmetall-Katalysatoren anorganisch geträgerte Katalysatoren darstellen, sind Ru-Titandioxid, Ru-Siliziumdioxid, Ru-Aluminiumdioxid, Ru-Kohlenstoff, P d-Kohlenstoff, Pd-Titandioxid, Pd-Siliziumdioxid, Pd-Aluminiumdioxid, Rh-Titandioxid, Rh-Siliziumdioxid, Rh-Kohlenstoff, Ir-Kohlenstoff, Ir-Titandioxid, Pt-Kohlenstoff, Pt-Titandioxid, Pt-Aluminiumoxid und/ oder Pt-Siliziumdioxid bevorzugt. Ganz besonders bevorzugt werden als Übergangsmetall-Katalysatoren Pd-Kohlenstoff, P d-Titandioxid, Pd-Siliziumdioxid, Pd-Aluminiumdioxid, Pt-Kohlenstoff, Rh-Kohlenstoff und Ru-Kohlenstoff, ganz besonders bevorzugt Pd-Aktivkohle oder Pt-Aktivkohle eingesetzt.

Die Übergangsmetall-Katalysatoren können beispielsweise als makroskopischer Festkörper oder als Partikel z.B. in Form einer kolloidalen Lösung eingesetzt. Beispielsweise und vorzugsweise weisen die Übergangsmetall-Katalysatoren einen Wassergehalt von 1 - 90 %, besonders bevorzugt von 20 - 80 % und ganz besonders bevorzugt von 40 - 60 % auf.

Bei den eingesetzten geträgerten oder nicht geträgerten Übergangsmetallkatalysatoren handelt es sich um handelsübliche Produkte.

In dem erfindungsgemäßen Verfahren kann der Wasserstoff beispielsweise in elementarer Form der Reaktionsmischung zugeführt werden. Der Wasserstoff kann aber auch in situ aus anderen Wasserstoffquellen, wie beispielsweise Hydrazin, Wasserstoffperoxid, Lithiumalanat oder Natriumborhydrid hergestellt werden. Bevorzugt wird der Wasserstoff außerhalb der Reaktionsmischung erzeugt und zugeführt.

Viele der Verbindungen der Formel (II) sind handelsüblich. Die Herstellung der Verbindungen der Formel (II) kann nach analogen, aus dem Stand der Technik bekannten Verfahren erfolgen und ist dem Durchschnittsfachmann bekannt.

Die Herstellung der Verbindungen der Formel (II) kann aber beispielsweise und vorzugsweise auch so erfolgen, dass Verbindungen der Formel (III)

(ARYL-N)-Y (III)

wobei ARYL-N die vorgenannte Bedeutung besitzt und Y Brom, Chlor, Iod oder Pseudohalogen darstellt,
mit Verbindungen der Formel (IV)

[R²-O⁻] Kat⁺ (IV)

wobei Kat⁺ ein beliebiges einfach geladenes Kation oder ein 1/n-tes Äquivalent eines n-wertigen Kations darstellt, umgesetzt wird und R² die vorgenannte Bedeutung hat.

Beispiele für Verbindungen der Formel (IV) sind Natrium[2-trifluormethoxyphenolat], Natrium[trifluormethylphenolat], Natrium[4-trifluormethoxyphenolat] und Kalium[4-trifluormethylphenolat].

Pseudohalogen bezeichnet im Rahmen der Erfindung Reste, die in ihren chemischen Eigenschaften eine große Ähnlichkeit zu den Halogenen aufweisen. Dies sind z.B. Sulfonate und Halogensulfonate, wie z.B. Tosylat, Triflat, Mesylat und Nonafluorbutylsulfonat, aber auch Thiocyanat und Azid, wobei Halogen die vorgenannte Bedeutung hat.

In einer Ausführungsform der Erfindung liegt die Konzentration des Übergangsmetalls in den Übergangsmetall-Katalysatoren bezogen auf die eingesetzte Masse der Verbindungen der Formel (II), zwischen 0,01 und 10 Gew. %. Bevorzugt liegt die Konzentration zwischen 1 und 7 Gew. %. Besonders bevorzugt zwischen 2 und 5 Gew. %.

Die Menge der eingesetzten Übergangsmetall-Katalysatoren kann so gewählt werden, dass die Menge an Übergangsmetall, bezogen auf die Verbindungen der Formel (II), zwischen 0.0001 und 95 mol %, bevorzugt zwischen 0.001 und 10 mol % und besonders bevorzugt zwischen 0.002 und 1 mol % liegt.

Beispielsweise und vorzugsweise beträgt das Stoffmengenverhältnis der Verbindungen der Formel (II) und der organischen, polaren Lösungsmittel im Reaktionsgemisch zwischen 1 : 2 und 1 : 1000. Bevorzugt liegt das Stoffmengenverhältnis zwischen 1: 5 und 1: 80. Besonders bevorzugt zwischen 1 : 10 und 1: 40.

Die Reaktionstemperatur liegt vorzugsweise zwischen 20°C und 200°C, besonders bevorzugt zwischen 50°C und 150°C, ganz besonders bevorzugt zwischen 70°C und 100°C.

Die Reaktion wird vorzugsweise bei Drücken zwischen 1 und 200 bar besonders bevorzugt zwischen 20 und 80 bar, ganz besonders bevorzugt zwischen 30 und 50 bar durchgeführt.

In einer Ausführungsform der Erfindung wird zunächst die Verbindung der Formel (II) mit den organischen, polaren Lösungsmitteln in Kontakt gebracht und dann der Übergangsmetall-Katalysator zugegeben. Ebenfalls möglich ist auch die Übergangsmetall-Katalysatoren gleich zur Reaktionsmischung zuzugeben.

Bevorzugt wird die Reaktion in einem Autoklaven durchgeführt. Des Weiteren ist bevorzugt unter inerten Bedingungen zu arbeiten. Die Inertisierung kann z.B. durch kontinuierliche oder diskontinuierliche Durchströmung der Reaktionsmischung mit inerten Gasen, wie z.B. Argon und Stickstoff, durchgeführt werden. In diesem Fall wird anschließend Wasserstoff aufgedrückt und z.B. bis zur Druckkonstanz hydriert. Ebenso kann die Reaktion aber auch schon früher beendet werden und dadurch teilhydrierte Produkte hergestellt werden.

In einer besonders bevorzugten Ausführungsform wird die Verbindung der Formel (II) mit den Lösungsmitteln, insbesondere den Mono-, Di- oder Tricarbonsäuren, in Kontakt gebracht. Im Anschluss daran werden die Übergangsmetall-Katalysatoren zugegeben, der Autoklav bzw. die Reaktionsmischung inertisiert, das Reaktionsgemisch erwärmt und erst in der letzten Stufe Wasserstoff aufgedrückt und bis zur Druckkonstanz hydriert.

Auf erfindungsgemäße Weise können die Verbindungen der Formel (I) in sehr kurzer Zeit, d.h. sehr wirtschaftlich in hohen Ausbeuten in technischen Prozessen hergestellt werden. Die Aufarbeitung kann in an sich bekannter Weise, z.B. durch Extraktion mit bekannten Lösungsmitteln wie beispielsweise Natronlauge und Toluol erfolgen.

Die erfindungsgemäß hergestellten Verbindungen der Formel (I) eignen sich insbesondere als Zwischenprodukte z.B. zur Herstellung von Arzneimitteln und Agrochemikalien.

Die nachstehenden Beispiele dienen der Erläuterung der Erfindung, ohne dabei auf diese beschränkt zu sein.

### Beispiele

### 1. Herstellung von 4-[4-Trifluormethoxyphenoxy]piperidin

70 g 4-(4-Trifluormethoxyphenoxy)pyridin (0,27 mol) wurden in 362 g Essigsäure (6,03 mol) gelöst und in einem Autoklaven vorgelegt. 3,5 g eines 50% wasserfeuchten 10%igen Palladium auf Aktivkohle Katalysators (2,5 Gew. % bezogen auf die Masse in Gramm des eingesetzten 4-(4-Trifluormethoxyphenoxy)pyridin) wurden zugegeben und der Autoklav inertisiert. Die Reaktionsmischung wurde auf 90 °C erwärmt und anschließend Wasserstoff auf 40 bar aufgedrückt und bis zur Druckkonstanz hydriert. Die Reaktionsmischung wurde abgekühlt, der Autoklav entspannt und die Reaktionsmischung entnommen. Der Katalysator wurde über Kieselgur (Celite) abfiltriert und das Lösungsmittel unter vermindertem Druck entfernt. Das Rohprodukt wurde in Toluol aufgenommen und mit Natronlauge extrahiert, die Phasen getrennt und die organische Phase unter vermindertem Druck zur Trockne eingeengt.

Man erhielt 50 g 4-[4-Trifluormethoxyphenoxy]piperidin (0,19 mol) in einer Ausbeute von 69,8%.

### 2. Herstellung von 4-[4-Methoxyphenoxy]-N-piperidinylacetamid

20 g 4-(4-Methoxyphenoxy)pyridin (99 mmol) wurden in 400 g Essigsäure (6,66 mol) gelöst und in einem Autoklaven vorgelegt. 1,6 g eines 50% wasserfeuchten 10%igen Palladium auf Aktivkohle Katalysators (4 Gew. % bezogen auf die Masse in Gramm des eingesetzten 4-(4-Methoxyphenoxy)pyridin) wurden zugegeben und der Autoklav inertisiert. Die Reaktionsmischung wurde auf 90 °C erwärmt und anschließend Wasserstoff auf 40 bar aufgedrückt und bis zur Druckkonstanz hydriert. Die Reaktionsmischung wurde abgekühlt, der Autoklav entspannt und die Reaktionsmischung entnommen. Vom Katalysator wurde abfiltriert und das Lösungsmittel unter vermindertem Druck entfernt. Das Rohprodukt wurde in Toluol aufgenommen und mit Natronlauge extrahiert, die Phasen getrennt und die organische Phase unter vermindertem Druck zur Trockne eingeengt.

Man erhielt 19,8 g 4-[4-Methoxyphenoxy]-*N*-piperidinylacetamid (79 mmol) in einer Ausbeute von 80%.

## Patentansprüche

1. Verfahren zur Herstellung von Verbindungen der Formel (I)
[R²-O]-ZYKLYL-N-R¹ (I)
**dadurch gekennzeichnet, dass** Verbindungen der Formel (II)
[R2-O]-ARYL-N (II)
in Gegenwart von mindestens einem Übergangsmetall-Katalysator,
mindestens einem organischen polaren Lösungsmittel
und Wasserstoff
umgesetzt werden,
wobei ZYKLYL-N für einen gegebenenfalls substituierten 5- bis 8- gliedrigen, zyklischen gesättigten oder teilweise ungesättigten, nicht aromatischen, N-heterozyklischen Rest, mit bis zu zwei Stickstoffatomen steht, der über ein Ringkohlenstoffatom mit dem Sauerstoffatom des R²-O-Restes verknüpft ist, und ARYL-N für einen gegebenenfalls substituierten N-heterozyklischen 5- bis 8- gliedrigen aromatischen Rest, mit bis zu zwei Stickstoffatomen steht, der über ein Ringkohlenstoffatom des aromatischen Restes mit dem Sauerstoffatom des R²-O-Restes verknüpft ist und R² für ein gegebenenfalls substituiertes C₆-C₂₀-Aryl steht und
R¹ mit einem Stickstoffatom des ZYKLY-N verbunden ist und Wasserstoff oder -C(=O)(R) darstellt und R für C₁-C₁₅-Alkyl, C₁-C₈-Alkylthio, C₁-C₈-Alkoxy, C₆-C₂₄-Aryl, C₈-C₂₆-Arylalkyl, C₁-C₈-Halogenalkyl, C₁-C₈-Halogenalkoxy, C₁-C₈-Halogenalkylthio, mit Halogen = F, Cl, Br, 5- bis 8-gliedriges gesättigtes Heterozyklyl mit Hetero = (Sauerstoff, Schwefel, oder Stickstoff), Wasserstoff oder C₁-C₈-Mono- bzw. C₁-C₁₆-Dialkylamino, die gegebenenfalls weiter substituiert sein können, steht.

2. Verfahren nach Anspruch 1, **dadurch gekennzeichnet, dass** R² für einen C₆-C₂₀-Arylrest, und/oder gegebenenfalls substituierten (C₁-C₆)-Alkyl, (C₁-C₆)-Alkoxy, (C₁-C₇)-Perfluoralkyl, (C₁-C₇)-Perchloralkyl, (C₁-C₇)-Perfluoralkoxy und/oder (C₁-C₇)-Perchloralkoxyrest steht.

3. Verfahren nach einem der Ansprüche 1 oder 2, **dadurch gekennzeichnet, dass** ARYL-N für Pyridyl, Pyrimidinyl, Imidazolyl und/oder Pyridazinyl steht, wobei diese gegebenenfalls mit (C₁-C₆)-Alkyl-, (C₁-C₆)-Alkoxy-, (C₁-C₇)-Perfluoralkyl-, (C₁-C₇)-Perchloralkyl-, (C₁-C₇)-Perfluoralkoxy-und/oder (C₁-C₇)-Perchloralkoxyresten substituiert sind.

4. Verfahren nach einem oder mehreren Ansprüchen 1 bis 3, **dadurch gekennzeichnet, dass** es sich bei dem organischen polaren Lösungsmittel um aliphatische oder alizyklische Ketone, Ether, Anhydride, Ester und/oder Carbonsäuren handelt.

5. Verfahren nach Anspruch 4, **dadurch gekennzeichnet, dass** die organischen, polaren Lösungsmittel Methansäure, Ethansäure, Propansäure, n-, t- , i - Butansäure, Isopropansäure, Pentansäure, Butandisäure, Oxalsäure, Zitronensäure und/oder Pyrrolidin-2-carbonsäure sind.

6. Verfahren nach einem oder mehreren der Ansprüche 1 bis 5, **dadurch gekennzeichnet, dass** das Übergangsmetall des Übergangsmetall-Katalysators Rhodium, Ruthenium, Platin und/oder Palladium ist.

7. Verfahren nach Anspruch 6, **dadurch gekennzeichnet, dass** der Übergangsmetall-Katalysator Ru-Titandioxid, Ru-Siliziumdioxid, Ru-Aluminiumdioxid, Ru-Kohlenstoff, Pd-Kohlenstoff, Pd-Aktivkohle, Pd-Titandioxid, Pd-Siliziumdioxid, P d-Aluminiumdioxid, Rh-Titandioxid, Rh-Siliziumdioxid, Rh-Kohlenstoff, Ir-Kohlenstoff, Ir-Titandioxid, Pt-Kohlenstoff, Pt-Titandioxid, Pt-Aluminiumoxid und/oder Pt-Siliziumdioxid ist.

8. Verfahren nach einem oder mehreren der Ansprüche 1 bis 7, **dadurch gekennzeichnet, dass** sich das Übergangsmetall auf einem Träger befindet, der aus Siliziumdioxid, Aluminiumoxid, Titandioxid, Zeolith und/oder Kohlenstoff besteht.

9. Verfahren nach einem oder mehreren der Ansprüche 1 bis 8, **dadurch gekennzeichnet, dass** die Verbindungen der Formel (I) für 4-[4-Trifluormethoxyphenoxy]piperidin, 4-[4-Methylphenoxy]piperidin, 4-Phenoxypiperidin, 4-[4-Methoxyphenoxy]piperidin, 4-[2-Trifluormethoxyphenoxy]piperidin, 4-[4-Trifluormethoxyphenoxy]-N-piperidinylacetamid, 4-[4-Methyl-phenoxy]-N-piperidinylacetamid, 4-Phenoxy-N-piperidinylacetamid, 4-[4-Methoxy-phenoxy]-N-piperidinylacetamid und 4-[2-Trifluormethoxyphenoxy]-N-piperidinylacetamid stehen.

10. Verfahren nach einem oder mehreren der Ansprüche 1 bis 9, **dadurch gekennzeichnet, dass** der Wasserstoff in situ erzeugt wird.

11. Verfahren nach einem oder mehreren der Ansprüche 1 bis 10, **dadurch gekennzeichnet, dass** die Reaktionstemperatur zwischen 20 °C und 200 °C liegt.

12. Verfahren nach einem oder mehreren der Ansprüche 1 bis 11, **dadurch gekennzeichnet, dass** der Reaktionsdruck zwischen 1 und 200 bar liegt.

13. Verfahren nach einem oder mehreren der Ansprüche 1 bis 12, **dadurch gekennzeichnet, dass** die Menge des Übergangsmetalls, bezogen auf die Verbindungen der Formel (II), zwischen 0.001 und 95 mol % liegt.

14. Verfahren nach einem oder mehreren der Ansprüche 1 bis 13, **dadurch gekennzeichnet, dass** das Stoffmengenverhältnis der Verbindungen der Formel (II) zu dem organischen, polaren Lösungsmittel zwischen 1: 5 und 1: 80 liegt.

15. Verwendung der Verbindungen der Formel (I) als Zwischenprodukte für die Herstellung von Arzneimitteln und Agrochemikalien.
